# EUROPEAN PATENT SPECIFICATION

(11) **EP 1 633 211 B1**
(45) Date of publication and mention of the grant of the patent: **16.06.2010**
(21) Application number: 03741645.0
(22) Date of filing: 18.06.2003
(51) Int. Cl.: A23L 2/40, A23L 1/05, A23L 1/308, A61K 9/00, A23L 1/0524, A23L 1/0532, A61K 31/732, A61K 31/734, A61K 31/715, A23L 2/385

(54) **HOT LIQUID FIBER PRODUCT**
HEISSES FLÜSSIGES FASERPRÄPARAT
PRODUIT FIBREUX LIQUIDE CHAUD

(43) Date of publication of application: 15.03.2006
(73) Proprietor: N.V. Nutricia, 2712 HM Zoetermeer (NL)
(72) Inventor: VAN LAERE, Katrien, Maria, Josefa, 6666 WS Heteren (NL); RAGGERS, Rene, John, 1057 NX Amsterdam (NL); ELBERSE, Johanna, Maria, Martina, 6707 AM Wageningen (NL)
(74) Representative: van Westenbrugge, Andries
(86) International application number: PCT/NL2003/000445
(87) International publication number: WO 2004/110178

(56) References cited:
- EP-A- 0 455 475
- FR-A- 2 733 420
- US-A- 1 945 963
- US-A- 4 414 198
- US-A- 5 476 675
- US-B1- 6 290 997

## Description

### TECHNICAL FIELD

The present invention relates to a process for the preparation of a hot liquid fiber product which can suitably be used in a method for the treatment and/or prevention of diet responsive conditions, e.g. overweight.

### BACKGROUND OF THE INVENTION

Fiber fortified drinks have been used for several purposes, e.g. for the treatment of diarrhea, for the treatment of obesity and as medicine carriers.

Stillman, in US6248390, describes a shelf stable, ready to use, essentially tasteless and odorless water-like fluid for humans/animals comprised of safe water and a significant quantity of one or more water-soluble dietary fibers. Fiber-water is intended to be consumed by drinking or by enteral feeding alone, and/or in combination. The liquid may be consumed directly hot or cold or after use, at any required temperature, in the preparation/reconstitution of beverages or liquid food product (e.g. coffee, tea, concentrates such as "HAWAIIAN PUNCH(R)", frozen concentrates such as lemonade/orange juice, soups and pet food).

WO02/096219 (Wolf et al) describes a method of blunting the postprandial glycemic response to a meal by feeding an acid controlled induced viscosity fiber system. The first component of the induced viscosity fiber system is anionic soluble fiber. The second component of the induced viscosity fiber system is water-insoluble, acid-soluble multivalent cations.

Tarral et al (WO96/33694) describes pectic preparations which comprise at least one pectin associated with a complex composition comprising firstly an effervescent pair which easily disperses the pectin in water and hydrates it and regulates the gellification process irrespective of the hardness of the water used for the suspension, and secondly a mixture of compounds which provide the calcium ions necessary for the formation of the gel in an acid medium, together with magnesium ions regulating the kinetics of the calcium availability.

In WO99/59542, Tarral et al describes a pectic preparation useful as medicament carrier, to be orally administered and capable of forming a gel in an acid medium, comprising at least a pectin having a degree of methoxylation higher than 15% and calcium ions, containing a gelling process inhibitor with a pH higher than 6.

The liquid fiber products described in the above prior art publications have in common that they suffer from unsatisfactory palatability.

US 1,945,963 teaches about improved pectin preparations capable of being readily and rapidly dispersed in aqueous media without the formation of lumps or undesirable frothing.

US 4,414,198 concerns a rapidly water-disintegrable tablet comprising a disintegrable system comprising an unreacted, intimate mixture of alginic acid and a water soluble metal carbonate in proportions reactive to form metal alginic acid salt and carbonic acid when the tablet is placed in water.

US 6,290,997 discloses a ready-to-drink beverage which is preferably frothy and foamy with a high flavor impact at a low dosage of solids.

US 5,476,675 discloses a process for preparing a dry edible flavor fiber product by admixing water, a powdered flavor, a binder and fiber together and extruding the resulting mixture.

### SUMMARY OF THE INVENTION

High fiber drinks known in the art may be prepared by a consumer by reconstituting a fiber containing powder in a suitable liquid, e.g. water. However, simply mixing the fiber containing powder often gives rise to problems, e.g. the formation of aggregates (lumps). Formation of aggregates may be reduced by vigorous shaking or stirring. However, this in turn may result in undesired foaming of the drink. Furthermore, fiber drinks made by reconstitution with water often suffer from bad palatability, which may at least partially be caused by the high viscosity and/or aggregates in these fiber containing drinks.

The inventors have found that the palatability can be improved by inclusion of an effervescent system in a fiber containing reconstitutable powder. However, they also observed that this did not improve palatability sufficiently. The inventors surprisingly discovered that the palatability (e.g. mouthfeel) of the fiber drink was significantly improved if a reconstitutable fiber-effervescent mix is reconstituted with hot water.

### DETAILED DESCRIPTION

In one aspect, the present invention relates to a process for preparing a hot liquid fiber product for enteral administration, comprising admixing:
a. a serving of a reconstitutable composition comprising
   (i) between 0.1 gram and 75 grams pectin;
   (ii) indegestible oligosaccharide with a degree of polymerization exceeding 2 and below 60 monose units ; and
   (iii) an effervescent system;
      and further comprising a calcium salt, and
b. a liquid with a temperature that exceeds 35°C

In a further aspect, the present invention relates to a packaged reconstitutable composition which bears instructions to mix the contents with a liquid having a temperature above 35°C, said composition comprising per serving:
(i) between 0.1 gram and 75 grams pectin;
(ii) indegestible oligosaccharide with a degree of polymerization exceeding 2 and below 60 monose units ; and
(iii) an effervescent system
(iv) and a calcium salt.

### Terms

The term "fiber" or "indigestible fiber" as used in the present invention refer to carbohydrates that are indigestible by human enzymes and acids present in the human intestinal tract.
The term "soluble fiber" refers to fibers that are soluble in aqueous solutions, particularly water soluble fiber.
The term "reconstitutable composition" is hereinafter abbreviated to RC. In a preferred embodiment, the RC is packaged and accompanied with instructions to admix the RC with a liquid having a temperature above 35°C. Preferably, the packaging bears (written) instructions to mix the RC with a liquid having a temperature above 35°C.

### Fiber

The present RC, which can be suitably used in the present process, preferably contains between 0.1 and 75 grams pectin per serving. It was found that a particularly good balance betwee palatability and functionality is achieved if the amount of pectin is in the range of between 0.25 and 35 grams, especially if said range is between 0.5 and 15 grams, even more preferably between 0.75 and 10 grams per serving, most preferably between 1 and 7 grams. Preferably low methoxylated pectin is used.

Preferably the RC contains between 0.1 and 10 grams, more preferably between 0.25 and 5 grams, most preferably between 0.5 and 4 grams of at least one fiber selected from the group consisting of low and high methoxylated pectin, preferably low methoxylated pectin.
Preferably the RC contains between 0.1 and 10 grams, more preferably between 0.2 and 7 grams, even more preferably between 0.25 and 5 grams low methoxylated pectin a per serving.

Preferably the pectin has an average molecular weight of at least 400 Da, more preferably of at least 5000 Da, even more preferably of at least 12000 Da, most preferably of at least 15000 Da.

### Effervescent system

In order to ensure proper dispersion of the fiber into the hot liquid, without the occurrence of significant formation of aggregates, the present reconstitutable composition contains an effervescent system. The amount of effervescent system in the RC is preferably limited so as to prevent excessive effervescent action.
The effervescent system comprises at least a base that liberates carbon dioxide and/or a base that liberates oxygen. Preferably, the effervescent system comprises a base that liberates carbon dioxide when it is contacted with added hot water. The effervescent system may be a single base that liberates carbon dioxide. The base that liberates carbon dioxide is preferably selected from the group consisting of carbonates, sesquicarbonates and hydrogencarbonate salts of potassium, lithium, sodium, calcium, ammonium, L-lysine carbonates, arginine carbonates, sodium glycine carbonates, sodium amino acid carbonates and combinations thereof. Preferably the effervescent system contains a carbonate salt, more preferably a carbonate salt selected from the group consisting of sodium carbonate, potassium carbonate, calcium carbonate, sodium bicarbonate, potassium bicarbonate, calcium bicarbonates, ammoniumbicarbonate, sodium glycine carbonate and combinations thereof. The use of only calcium carbonate may not provide sufficient dispersion of the ingredients in the hot water. Hence, in a particularly preferred embodiment, the present reconstitutable composition comprises at least one salt selected from the group consisting of sodium carbonate, potassium carbonate, sodium bicarbonate, potassium bicarbonate, calcium bicarbonates, ammonium bicarbonate and sodium glycine carbonate; optionally in combination with calcium carbonate. Most preferably the base that liberates carbon dioxide comprises at least one bicarbonate salt, which is preferably selected from the group consisting of calcium bicarbonate, potassium bicarbonate and sodium bicarbonate.

The present RC preferably contains between 0.05 and 10 grams base that liberates carbondioxide (preferably carbonate salt) per serving, more preferably between 0.1 ad 5 grams per serving, even more preferably at least 0.2 grams per serving, most preferably between 0.25 and 3 grams per serving.

The concentration of the base that liberates carbon dioxide in the RC is preferably between 0.5 and 80 wt.%, more preferably 1 and 50 wt.%, more preferably between 2 and 40 wt.% by weight.

Optimising the weight ratio of fiber:base that liberates carbon dioxide is important to avoid both foaming and lumping. In a preferred embodiment the present RC has a weight ratio fiber:base of about 100:1 1 to 1:10, more preferably of about 10:1 1 to 1:2 , even more preferably from about 10:1 1 to 1:1.

In order to facilitate dispersion and to further reduce formation of aggregates the present composition preferably comprises a carrier material which prevents the fibers from forming a gel matrix. Hence, the fiber content of the present reconstitutable composition is preferably below 90 wt.% based on the total dry weight of the RC, more preferably below 75 wt.%.

Preferably, the effervescent system comprises an effervescent couple, comprising the base that liberates carbon dioxide as well as an acidic component. The term "acidic component" refers to any compound or material that can serve as a proton source and that can react with the base to liberate carbon dioxide.
The acidic component can have more than one acid dissociation constant, i.e. comprise more than one acid functional group. The acidic component can be any organic or inorganic acid in the free acid, acid anhydride or acid salt form. According to a preferred embodiment the acidic component is solid at room temperature and a saturated solution of the acidic component exhibits pH 4.5 or lower at room temperatures. The acidic component may also suitably be an acid alkali metal salt of the latter acidic component that is solid at room temperature (e.g. a sodium salt, potassium salt, etc.). As the acidic component a compound which is not harmful to animals including man is desirably employed.
The acidic component can be tartaric acid, citric acid, maleic acid, fumaric acid, malic acid, adipic acid, succinic acid, lactic acid, glycolic acid, alpha hydroxy acids, ascorbic acid, amino acids and their alkali hydrogen acid salts. In case of an acid substance such as phosphoric acid or pyrophosphoric acid or other inorganic acids which are liquid at room temperature, an acid alkali metal salts that is solid at room temperature may suitably be employed.
The acidic component of the effervescent couple preferably comprises a solid physiologically acceptable acid selected from the group consisting of citric, tartaric, adipic, fumaric, maleic, malic, lactic acetic and benzoic acids and phosphates, more preferably from the group selected from citric, tartaric, adipic, fumaric or malic acids.

To reduce the occurrence of foaming in the present process and to prevent the liquid fiber product from developing a high viscosity during admixing with hot water, the effervescent couple preferably contains limited amounts of acidic component, e.g. preferably less than 10 grams, preferably less than 5 grams, even more preferably less than 2 grams, most preferably less than 1 gram acidic component per serving. Preferably the present RC contains at least 0.025 g acidic ingredient. The preferred level of the acidic component and base that liberates carbondioxide, is between about 0.05 and 10 grams, preferably between 0.1 and 5 grams, even more preferably between 0.25 and 3 grams of base that liberates carbon dioxide (e.g. a bicarbonate salt) per serving; and between 0.05 and 10 gram, more preferably between 0.1 and 5 grams, even more preferably between 0.5 and 3 grams acidic component (such as citric acid) per serving.

### Calcium

According to the invention the present RC contains calcium salt. The combination of calcium with pectin that gels in the presence of calcium (e.g. low methoxylated pectin provides good satiation, making the liquid fiber product particularly useful in a method for the treatment and/or prevention of diet responsive conditions such as overweight.
Generally, such fiber products display a relatively high viscosity due to the gel-forming reaction between the calcium and the pectin. A known measure to reduce the viscosity of the fiber product is to employ insoluble calcium salt in a neutral or high pH liquid, which insoluble calcium salt will dissolve as a result of pH reduction and/or temperature increase. However, such products do not exhibit a satisfactory mouthfeel and generally have a high viscosity. Also it is generally difficult to dissolve these pectin/calcium systems. The use of hot water to improve solubility is generally to be avoided as this will lead to the formation of lumps, possibly due to Ca-induced gellation.
The inventors have surprisingly found that calcium/pectin systems may suitably be used in the present RC as the resulting liquid fiber product has excellent mouthfeel and palatability. The effervescent system in the RC was found to prevent the formation of lumps in a surprisingly effective way, even if very hot water is used to dissolve the RC. In a preferred embodiment, the present invention relates to a RC comprising low methoxylated pectin, calcium salt and an effervescent couple. The admixture of these pectin, calcium and effervescent couple with hot water results in a liquid fiber product with a surprisingly low viscosity, presumably because the base that liberates carbon dioxide more rapidly neutralizes the acidic component and more quickly disperses the fiber, than is the case if the water is at room temperature.
The present RC preferably contains a calcium salt, which is substantially less soluble in water at 37°C and at the pH of the present composition than at 37°C and a pH below 5. Such a calcium salt, when present in the composition in an amount that exceeds its maximum solubility, will dissolve in the stomach under the influence of pH-reduction and/or temperature increase. Thus the calcium ion concentration in the stomach will increase, which will automatically stimulate pectin gellation.

Because of the gellation inducing effect of calcium ions, the concentration of such ions in the present liquid composition (at near neutral pH) is preferably relatively low. The limited presence of (dissolved) calcium ions at around neutral pH prevents the formation of a gel matrix which would impart unacceptably high viscosity. Thus, in a preferred embodiment, the calcium salt(s) used in the present composition have a solubility below 0.15, more preferably below 0.1, even more preferably below 0.06 gram per 100 ml (demineralised) water at 20°C and pH 7.

Preferably, the calcium salt(s) provide more than 0.05 gram dissolved calcium per 100 ml water at a pH below 4 and a temperature of 37°C, more preferably it provides more than 0.1 g per 100 ml water under these conditions. The solubility of the calcium salt at pH below 4 and at 37°C is not bound to an upper limit, however, is typically below 50 g of the salt per 100 ml water.

The calcium salt is preferably selected from the group consisting of calcium phosphate (e.g. tribasic, dibasic, monobasic or penta calcium triphosphate), calcium carbonate, calcium sulfate, calcium oxide, calcium citrate (e.g. mono-calcium citrate or tri-calcium citrate), a calcium salt coated with a substance which has limited solubility in water at pH 7 and is soluble at a pH below about 5 (hereafter referred to as coated calcium salts) and mixtures thereof. Examples of coatings and methods for the preparations of coated calcium salts are given in WO0038829.

More preferably, the calcium salt is selected from the group consisting of coated calcium salt, calcium carbonate and mixtures thereof. Even more preferably between 50 wt.% and 100 wt.% of calcium salt is provided as calcium carbonate.

To provide optimal gelling characteristics without significantly effecting the palatability of the liquid fiber product, the RC preferably contains less than 10 wt.% calcium salt, more preferably less than 5 wt.% calcium salt, even more preferably less than 2.5 wt.% calcium salt, most preferably less than 1 wt. % calcium salt based on the total weight of the RC.
The RC preferably comprises between 10 mg and 5 grams, preferably between 25 mg and 2.5 grams, even more preferably between 0.50 and 1 gram calcium per serving.

The liquid composition prepared with the present process preferably contains at least 25 mg Ca per liter. Preferably the calcium concentration exceeds 50 mg Ca per liter, more preferably it exceeds 100 mg Ca per liter, most preferably it exceeds 150 mg Ca per liter. Furthermore, the calcium concentration in the composition preferably does not exceed 15 gram Ca per liter, more preferably it does not exceed 5 gram Ca per liter, even more preferably it does not exceed 3 gram Ca per liter.

### Liquid fiber product

Advantageously the viscosity of the liquid product, obtainable with the present process and/or from the present RC, and used advantageously in a method for the treatment and/or prevention of diet responsive conditions (e.g. overweight) has a viscosity below 100 mPas, more preferably below 50 mPas, even more preferably below 25 mPas, even more preferably below 10 mPas, most preferably below 5 mPas at a shear rate of 100s⁻¹. At pH 3 and 37°C, (at least part of) the product preferably has a viscosity which exceeds 200 mPas, more preferably exceeds 500 mPas, most preferably exceeds 1000 mPas at a shear rate of 100s⁻¹. Preferably, the viscosity of the composition at pH 3 and 37°C does not exceed 100,000 mPas at a shear rate of 100s⁻¹.

Whenever the term viscosity is used in the present document, this refers to the physical parameter may be determined using a Carri-Med CSL rheometer. The used geometry is of conical shape (6 cm 2 deg acrylic cone) and the gap between plate and geometry is set on 55 µm. A linear continuous ramp shear rate is used from 0 to 200 s⁻¹ in 30 seconds.
The rheometer's thermostat is set on the appropriate temperature.

In order to determine the viscosity at acidic pH (pH 3), first a sufficient amount of 1M HCl is homogeneously admixed (drop-wise under very gentle stirring for about 20 sec, to prevent the breakdown of the gel) to the liquid composition to adjust the pH of the composition to pH 3. Thereafter the composition is left standing at 37°C for about 10 minutes. Subsequently the composition thus obtained is used to determine the viscosity at pH 3, according to the method described above.

Preferably, the liquid fiber product, which is suitably prepared with present RC, is ingested at a temperature which exceeds 35°C, even more preferably at a temperature of about 37°C or above (e.g. about 37°C).

The liquid fiber product preferably has a pH which exceeds 3, more preferably exceeds 4, even more preferably exceeds 5, most preferably exceeds 6.

### Reconstitution liquid

The present invention relates to a process, which comprises admixing a powder with a hot liquid. Whenever the term hot is used, this refers to a liquid with a temperature above about 35°C, preferably a liquid with a temperature of about 37°C or above. The temperature of the liquid preferably does not exceed its boiling temperature, i.e. for water the temperature of the liquid preferably does not exceed 100°C.
Preferably the present RC is admixed with water having a temperature of between 35°C and 100°C, more preferably with water having a temperature between 50°C and 100°C.
The present packaged reconstitutable composition which bears instructions to mix the contents with a liquid having a temperature above 35°C, need not disclose the temperature indications literally, but may also refer to warm liquid, cooking water, steaming liquid and the like. Such terminology is generally interpreted as liquid with a temperature of about 35°C or above.

Preferably, the liquid that is admixed with the RC has a pH above about 3, more preferably above 4, even more preferably above 6. The liquid may be slightly acidic, because the effervescent system may reduce the acidity of the liquid. The reconstitution liquid preferably has a pH below about 10, even more preferably below about 8.

### Oligosaccharide

The RC further contains indigestible oligosaccharide.
The term "indigestible oligosaccharides" as used in the present invention refers to saccharides which have a degree of polymerisation of monose units exceeding 2, more preferably exceeding 3, most preferably exceeding 4, which are not or only partially digested in the intestine by the action of acids or digestive enzymes present in the human upper digestive tract (small intestine and stomach) but which are fermented by the human intestinal flora.
The degree of polymerisation of the oligosaccharide is below 60 monose units, preferably below 40, even more preferably below 20, most preferably below 10.
Suitable oligosaccharides are preferably fermented by the gut flora. The oligosaccharide used in the composition according to the present invention is preferably capable of significantly increasing the total cecal and/or colonic SCFA content.
Preferably the oligosaccharide is selected from the group consisting of:
cellobiose (4-O-β-D-glucopyranosyl-D-glucose), cellodextrins ((4-O-β-D-glucopyranosyl)ₙ-D-glucose), B-cyclodextrins (Cyclic molecules of α-1-4-linked D-glucose; α-cyclodextrin-hexamer, β-cyclodextrin-heptamer and γ-cyclodextrin-octamer), indigestible dextrin , gentiooligosaccharides (mixture of β-1-6 linked glucose residues, some 1-4 linkages), glucooligosaccharides (mixture of α-D-glucose), isomaltooligosaccharides (linear α-1-6 linked glucose residues with some 1-4 linkages), isomaltose (6-O-α-D-glucopyranosyl-D-glucose); isomaltriose (6-O-α-D-glucopyranosyl-(1-6)-α-D-glucopyranosyl-D-glucose), panose (6-O-α-D-glucopyranosyl-(1-6)-a-D-glucopyranosyl-(1-4)-D-glucose), leucrose (5-O-α-D-glucopyranosyl-D-fructopyranoside), palatinose or isomaltulose (6-O-α-D-glucopyranosyl-D-fructose), theanderose (O-α-D-glucopyranosyl-(1-6)-O-α-D-glucopyranosyl-(1-2)-B-D-fructofuranoside), D-agatose, D-*lyxo*-hexulose, lactosucrose (O-β-D-galactopyranosyl-(1-4)-O-α-D-glucopyranosyl-(1-2)-β-D-fructofuranoside), α-galactooligosaccharides including raffinose, stachyose and other soy oligosaccharides (O-α-D-galactopyranosyl-(1-6)-α-D-glucopyranosyl-β-D-fructofuranoside), β-galactooligosaccharides or transgalacto-oligosaccharides (β-D-galactopyranosyl-(1-6)-[β-D-glucopyranosyl]ₙ-(1-4) α-D glucose), lactulose (4-O-β-D-galactopyranosyl-D-fructose), 4'-galatosyllactose (O-D-galactopyranosyl-(1-4)-O-β-D-glucopyranosyl-(1-4)-D-glucopyranose), synthetic galactooligosaccharide (neogalactobiose, isogalactobiose, galsucrose, isolactoseI, II and III), fructans - Levan-type (β-D-(2→6)-fructofuranosyl)ₙ α-D-glucopyranoside), fructans - Inulin-type(β-D-((2→1)-fructofuranosyl)ₙ α-D-glucopyranoside), 1 f-β-tructofuranosylnystose (β-D-((2→1)-fructofuranosyl)ₙ B-D-fructofuranoside), xylooligosaccharides (B-D-((1→4)-xylose)ₙ, lafinose, lactosucrose, arabinooligosaccharides and mixtures thereof.
According to a further preferred embodiment the oligosaccharide is selected from the group consisting of fructans, fructooligosaccharides, indigestible dextrins galactooligosaccharides (including transgalactooligosaccharides), xylooligosaccharides, soybean oligosaccharides, arabinooligosaccharides, glucooligosaccharides, mannooligosaccharides, fucooligosaccharides and mixtures thereof.

The RC preferably contains between 0.1 and 100 grams, more preferably between 0.5 and 50, even more preferably between 1 and 25 grams oligosaccharides per serving.

The present liquid fiber product preferably includes at least 0.1 wt.%, preferably at least 0.2 wt.%, more preferably at least 0.5 wt.% and even more preferably at least 0.9 wt.% indigestible oligosaccharides. Although the administration of considerable amounts of oligosaccharides will generally not lead to undesirable side effects, the present composition preferably has an oligosaccharide content below 20 wt.%, more preferably below 10 wt.% even more preferably below 5 wt.%.

### Palatability

It is recognized that additional ingredients may be added to the formulation, such as flavouring agents, colouring agents, sweetening agents, antioxidants, stabilizers and/or processing aids.

### Serving

The term "serving" as used herein refers to a portion of the present RC, which is (after reconstitution) delivered, or meant to be delivered to a subject in a single administration event. In a particularly preferred embodiment, the present RC is packaged in serving lots. The RC may also be packaged in a container containing multiple servings, wherein said container bears instruction on how to separate a serving unit from the bulk. In the present process the RC is preferably admixed with a liquid having a volume of between 50 and 1000 ml, more preferably a volume of between 100 and 500 ml, even more preferably a volume of between 120 and 400 ml, most preferably between 150 and 350 ml, to prepare a proper serving.

### Low caloric liquid fiber product

In a preferred embodiment, the present invention relates to low caloric RC, which results, after admixing hot water, in a hot, low caloric liquid fiber product. The fiber in the present low caloric RC is preferably low methoxylated pectin.

One serving of the present low caloric RC preferably contains between 0 and 200 kcal, more preferably between 0 and 100 kcal, even more preferably between 0 and 50 kcal, most preferably between 0 and 25 kcal, particularly between 0 and 10 kcal.

A low caloric hot fiber liquid prepared with the present process preferably contains between 0 and 500 kcal per liter. Advantageously, the liquid composition has a caloric density below 400 kcal per liter, even more preferably below 250 kcal per liter, most preferably below 50 kcal per liter, particularly below 25 kcal per liter.

The present low caloric RC preferably includes no more than a limited amount of caloric ingredients. Hence, preferably the present composition contains:
- less that 5 wt.% protein based on the total weight of the RC, preferably less than 1 wt.%, more preferably less than 0.1 wt.%; and/or
- less than 10 wt.% glycerides (i.e. tri-, di- and/or monoglycerides) and/or fatty acids based on the total weight of the RC, preferably less than 1 wt.%, more preferably less than 0.1 wt.%; and/or
- less than 50 wt.% digestible carbohydrates based on the total weight of the RC, preferably less than 5 wt.%, more preferably less than 0.5 wt.%.

### Hot beverages

It was found that the present RC can suitable be used in the preparation of drinks that are normally consumed at elevated temperature, such as tea, cocoa and coffee. Hence, the RC may further comprises one ingredient selected from the group of cocoa, reconstitutable coffee and reconstitutable tea. The RC preferably comprises an ingredient selected from caffeine, theobromine and catechin.

### Complete nutrition

The present RC can also advantageously take the form of powdered reconstitutable preparations that provide a complete nutrition, e.g. including carbohydrates and/or protein, optionally combined with fat, e.g. infant formula and clinical foods. In such applications the RC is preferably admixed with a reconstitution liquid which has a temperature of about 35°C to about 75 °C, even more preferably between 35°C and 40°C.

Hence, in a further preferred embodiment, the present invention relates to a composition comprising protein in an amount of between 1 and 25 grams per serving, preferably between 2 and 15 grams per serving, more preferably 3 and 7 grams per serving; digestible carbohydrates in an amount of between 2 and 100 grams per serving, preferably in an amount of between 5 and 50 grams per serving even more preferably in an amount of between 10 and 25 grams per serving; fat in an amount of between 1 and 25 grams per serving, preferably in an amount of between 2 and 20 grams per serving, even more preferably in an amount of between 5 and 10 grams per serving; pectin and an effervescent systems and a calcium salt.
One serving of the present composition preferably provides between 50 and 750 kcal, more preferably between 75 and 500 kcal, even more preferably at least 100 kcal, even more preferably between 100 and 250 kcal.

### Savoury products

Often, the fortification of drinks with fiber, particularly the satiety inducing fibers, does not improve the palatability of the drink. Additionally, the present inventors have found that some of the effervescent salts have a salty aftertaste.
At least part of this problem is solved by the present invention. It was found that the taste of the product could be even further improved if the product has a "salty" taste, e.g. if the fiber-effervescent system is combined in a soup or bouillon. Hence, the present invention also provides a (packaged) reconstitutable salty product containing pectin and effervescent system, e.g. the low methoxylated pectin; calcium and effervescent couple.
In a preferred embodiment, the present invention provides a reconstitutable (e.g. low caloric) soup or bouillon product, containing the present effervescent -fiber system. Hence, in a further aspect, the present invention relates to the present RC further containing natrium chloride and/or glutamate. Preferably the composition contains sodium chloride in an amount of between 0.05 and 25 grams, even more preferably in an amount of between 0.1 and 10 grams, even more preferably in an amount of between 0.4 and 5 grams sodium chloride per serving; and/or between 0.05 and 5 grams, preferably between 0.1 and 2 grams glutamate per serving.
The salty composition further preferably comprises at least one selected from the group consisting of meat flavoring (e.g. chicken, beef, pork), fish flavoring, vegetable flavoring (e.g. tomato, mushroom, carrot, union soup flavoring) and yeast extract.
Also these salty products can be advantageously used in a method for the reduction or prevention of diet responsive conditions such as overweight.

### Treatment

The present invention also provides a method for the treatment or prevention of a diet responsive condition in a mammal. The term "diet responsive condition" as used herein refers to any disease or physical state that can be caused or grow worse by the ingestion of proteins, carbohydrates or fats. The present RC can be used for the preparation of a hot liquid product which can be suitably used in a method for the treatment and/or prevention of a condition selected from the group of diabetes, overweight, high cholesterol levels (e.g. hypercholesteremia), diarrhea and coronary heart disease.

The present method is particularly suitable for (prophylactically) treating obesity as the appetite reducing effects of the liquid fiber product prepared with the present process will usually lead to a reduction of body weight, a reduction of body weight increase or a reduction in production of body fat.

Another advantageous application of the method is its use for suppressing fluctuations in blood glucose levels, which is particularly beneficial for diabetics. Suppression of blood glucose fluctuations, and particularly the blood glucose 'peaks', is also of benefit for obese people as the resulting gradual absorption of carbohydrates usually leads to less body fat formation than is observed for rapid absorption of the same amount of carbohydrates.

Hence, the hot liquid fiber product, particularly the low-caloric, pectin fortified compositions containing low methoxylated pectin; calcium and effervescent couple, can be advantageously used in a method for the treatment and/or prevention of overweight and or diabetes, said method comprising administering to a monogastric mammal the liquid fiber product which is suitably prepared from the present reconstitutable powder and/or with the present process.

The term "overweight" as used in the present invention refers to a bodyweight and/or an adipose tissue mass that is above the desired bodyweight and/or desired adipose tissue mass. Obese monogastric mammals are considered overweight. Human subjects who have a body mass index above 25 most advantageously use the present method. The present method can thus suitably be used to reduce adipose tissue mass or prevent an increase in adipose tissue mass and to increase the ratio lean body mass to adipose tissue mass and/or the ratio muscle mass to adipose tissue mass.
The present method can both be used to treat or prevent overweight in therapy and/or for the cosmetic treatment or prevention of overweight, i.e. losing weight.

The liquid fiber product suitably prepared with the present process and/or by reconstitution of the present RC can be used advantageously in a method for the reduction of the appetite in a human, said method comprising administering to said human an appetite reducing amount of the above described composition. The present method is preferably used to prevent or treat overweight in humans, even more preferably human adolescents and children, e.g. a method for the treatment and/or prevention of childhood obesity. The present liquid fiber product can also be advantageously used in a method for inducing satiation, said method comprising administering to a human the above-described composition.
Furthermore, the present liquid fiber product reduces caloric intake of a meal when the product is consumed shortly before or during the same meal. Hence the present invention also provides a method for reducing the caloric intake of a meal, for prevention of ingesting excess calories when ingesting a meal and/or controlling daily caloric intake, said method comprising administering to a human an effective amount of the liquid fiber product as described above, shortly before or during the meal (i.e. breakfast, brunch, lunch, high tea or dinner).

In a further preferred embodiment, the RC is accompanied with instructions and/or indicia which teach the use of liquid composition in association with the diet responsive condition. The indicia or instruction may be in any form, such as language directing the user towards the intended use. For the purpose of weight loss the product may carry a name which points the consumer towards the intended use, e.g. "slimwater", "low caloric drink", "slimsoup", "warm&slim", "hot&lean", "leandrink" or the like. Also pictures or drawings may be used to direct the consumer towards the intended use, such as a "slim" "figure" or "balance". The indicia are preferably selected from the group consisting of alphabetic indicia, numeric indicia, color indicia, graphic indicia, and combinations thereof.

### EXAMPLES

### Example 1: Viscosity of low caloric pectin calcium drink.

A reconstitutable powder with ingredients as depicted in Table I (with added flavor and color) was admixed with about 300 ml water with a temperature of about 70°C or 20 °C. Subsequently the viscosity was determined. The hot drink has low viscosity and good palatability (Table II).

**Table I: Ingredients of one serving of the reconsitutabel powder:**

| Ingredient | Weight |
|---|---|
| Pectin Genu LM | 2.0 gr |
| CaCO3 | 0.25 gr |
| Dextrose | 4.0 gr |
| Malic acid | 0.4 gr |
| Citric acid | 0.30 gr |
| NaHCO3 | 1.25 gr |
| KHCO3 | 1.49 gr |
| Acesulfaam-K | 0.015 gr |
| Sucralose | 0.04 gr |

**Table II: Viscosity and pH of a liquid product prepared with the reconstitutable powder of Table I.**

| Temperature | Viscosity | pH |
|---|---|---|
| 20°C | 16.5 mPas | 6.9 |
| 70°C | 3.1 mPas | 7.8 |

### Example 2: Viscosity of low caloric pectin drink.

A reconstitutable powder with a composition as depicted in Table III (with added flavor and color) was admixed with about 300 ml water with a temperature of about 70°C or 20 °C. Subsequently the viscosity was determined. The hot drink has low viscosity and good palatability (Table IV).

**Table III: Ingredient of one serving of the reconsitutabel powder:**

| Ingredient | Weight |
|---|---|
| Pectin Genu LM | 2.0 gr |
| CaCO3 | 0.25 gr |
| Dextrose | 4.0 gr |
| Malic acid | 0.4 gr |
| Citric acid | 0.30 gr |
| NaHCO3 | 0.68 gr |
| KHCO3 | 0.98 gr |
| Acesulfaam-K | 0.015 gr |
| Sucralose | 0.04 gr |

**Table IV: Viscosity and pH of liquid prepared with the reconstitutable powder of Table I.**

| Temperature | Viscosity | pH |
|---|---|---|
| 20°C | 40 mPas | 6.7 |
| 70°C | 3 mPas | 7.2 |

### Example 3: Viscosity of low caloric pectin drink.

The effervescent formula according to example 2, further comprising 2 grams Fibersol 2^{™} (Matsutani Chemical industry Co., Japan).

### Example 4: Effervescent infant formula

A reconstitutable infant nutrition powder, to be mixed with one liter warm water of about 37°C. A serving of between 100 and 250 ml can be taken from the reconstituted liquid.

**Table V: Formula According to the Invention Concentration per Nutrient liter of formula**

| Ingredient | Amount/liter |
|---|---|
| Protein | 13.0-20.3 g |
| Fat | 24.0-38.2 g |
| Carbohydrate | 70.0-110 g |
| Calcium | 510-910 mg |
| Phosphorus | 390-600 mg |
| Magnesium | 50-100 mg |
| Chloride | 420-575 mg |
| Iron | 8-16 mg |
| Zinc | 5-8 mg |
| Copper | 500-1000 mcg |
| Iodine | 100-510 mcg |
| Manganese | 34-500 mcg |
| Vitamin | A 2000-3800 IU |
| Vitamin D | 400-500 IU |
| Vitamin E | 20-26 IU |
| Vitamin K | 55-200 mcg |
| Vitamin C | 60-200 mg |
| Thiamin | 405-4100 mcg |
| Riboflavin | 610-2000 mcg |
| Pyridoxine | 400-800 mcg |
| Vitamin B-12 | 1.7-6 mcg |
| Niacin | 7-15 mg |
| Folic Acid | 100-275 mcg |
| Pantothenic Acid | 3-7.5 mg |
| Biotin | 30-150 mcg |
| Taurine | 45-70 mg |
| Carnitine | 35-60 mg |
| Choline | 50-202 mg |
| Inositol | 30-100 mg |
| Low methoxylated pectin | 0.5-10 g |
| NaHCO₃ | 1-5 g |
| citric acid | 1-6 g |
| KHCO₃ | 1-5 g |

### Example 5: Effervescent infant formula

The effervescent infant formula according to example 3, wherein the low methoxylated pectin has been replaced by between 1 and 10 grams xanthane gum.

### Example 6: Effervescent infant formula

The effervescent infant formula according to example 4, further comprising between 0.5 and 5 grams calcium carbonate.

### Example 7: Low caloric weight loss bouillon (one serving)

Bovril™ Instant Bouillon Powder Sachet which bears instructions to mix the contents with boiling water, containing: Salt, Monosodium Glutamate, Reconstituted Beef Stock, Hydrolysed Plant Protein, Caramel, Sugar, Modified Corn Starch, Yeast extract, Spices, Flavour, Mono- and Diglycerides combined with 2 grams Pectin Genu LM, 0.25g CaCO3, 0.5 g citric acid, 1.25 g NaHCO₃, and 1.49 g KHCO₃.

### Example 8: Low Caloric Weight loss instant tea (one serving)

Instant Tea Powder Sachet which bears instructions to mix the contents with 200 ml steaming water, containing:
Calcium Carbonate (125 mg), Low methoxylated Pectin (1000 mg), Oligosacharides (2000 mg), water soluble Ilex paraguariensis extract (400 mg), water soluble Black Tea extract (300 mg), water soluble Red Bush extract (400 mg), citric acid (50 mg), malic acid 350 mg, 0.11 g NaHCO₃, and 0.17 g KHCO₃.

### Example 9: Fiber fortified food

A powder composition which bears instructions to mix the contents with 200 ml hot water, containing comprising:
10 grams Stimulance ™ (Nutricia, The Netherlands)
0.30 gr Citric acid
1.25 gr NaHCO3
1.49 gr KHCO3

## Claims

1. A process for preparing a hot liquid fiber product for enteral administration, comprising admixing:
a. a serving of a reconstitutable composition comprising
(i) between 0.1 gram and 75 grams pectin;
(ii) indigestible oligosaccharide with a degree of polymerization exceeding 2 and below 60 monose units; and
(iii) an effervescent system;
and further comprising a calcium salt, and
b. a liquid with a temperature that exceeds 35°C.

2. The process according to claim 1, wherein the indigestible oligosaccharide is selected from the group consisting of fructans, fructooligosaccharides, indigestible dextrins galactooligosaccharides (including transgalactooligosaccharides), xylooligosaccharides, soybean oligosaccharides, arabinooligosaccharides, glucooligosaccharides, mannooligosaccharides, fucooligosaccharides and mixtures thereof.

3. The process according any one of the preceding claims, wherein the liquid has a volume of between 50 and 1000 ml.

4. The process according to any one of the preceding claims, wherein the fiber is low-methoxylated pectin.

5. The process according to any one of the preceding claims, wherein the effervescent system contains at least one base that liberates carbon dioxide selected from the group consisting of sodium carbonate, potassium carbonate, sodium bicarbonate, potassium bicarbonate, calcium bicarbonates, ammonium bicarbonate and sodium glycine carbonate.

6. The process according to claim 5, further comprising an acidic ingredient selected from the group consisting of as citric, tartaric, adipic, fumaric, malic, lactic, acetic, maleic and benzoic acids, and phosphates.

7. The process according to any one of the preceding claims, wherein the reconstitutable composition further comprises NaCl and/or glutamate.

8. The process according to any one of the preceding claims, wherein the resulting liquid fiber product has a pH above 4.

9. The process according to any one of the preceding claims, wherein the reconstitutable composition contains a calcium salt with a solubility below 0.15 g per 100 ml water at 20°C and pH 7 which provides more than 0.05 gram dissolved calcium per 100 ml water at a pH below 4 and a temperature of 37°C, and the resulting liquid product (i) has a viscosity below 100 mPas, and-(ii) exhibits a viscosity above 250 mPas when it is acidified to pH 3.

10. A hot liquid fiber product with a viscosity below 100 mPas, a pH that exceeds 4 and a temperature of at least 35°C, prepared with the process according to claim 9.

11. A hot liquid fiber product with a viscosity below 100 mPas, a temperature of at least 35°C and a viscosity of at least 250 mPas when it is acidified to pH 3, said composition comprising (i) a calcium salt with a solubility below 0.15 g per 100 ml water at 20°C and pH 7 which provides more than 0.05 gram dissolved calcium per 100 ml water at a pH below 4 and a temperature of 37°C, (ii) per serving between 0.1 gram and 75 grams pectin, (iii) indigestible oligosaccharide with a degree of polymerization exceeding 2 and below 60 monose units.

12. Use of fiber in the manufacture of composition for use in a method for the treatment and/or prevention of a diet responsive condition in a monogastric mammal, said method comprising enterally administering to the mammal the composition of claim 10 or 11.

13. Use according to claim 12 wherein the diet responsive condition is overweight.

14. A packaged reconstitutable composition which bears instructions to mix one or more servings of the reconstitutable composition with a liquid having a temperature above 35°C, said composition comprising per serving:
(i) between 0.1 gram and 75 grams pectin;
(ii) indigestible oligosaccharide with a degree of polymerization exceeding 2 and below 60 monose units; and
(iii) an effervescent system
(iv) and a calcium salt.

15. The packaged composition according to claim 14, wherein the composition comprises per serving:
(i) between 0.5 and 15 g low methoxylated pectin and/or alginate;
(ii) between 0.01 and 25 grams base that liberates carbon dioxide, preferably selected from the group consisting of sodium carbonate, potassium carbonate, calcium carbonate, sodium bicarbonate, potassium bicarbonate, calcium bicarbonates, ammonium bicarbonate and sodium glycine carbonate;
(iii) between 0.025 and 5 grams acidic ingredient, preferably selected from the group consisting of citric, tartaric, adipic, fumaric, malic acids, lactic, acetic, maleic and benzoic acids; and phosphates and
(iv) between 0.05 and 5 grams calcium.

## Patentansprüche

1. Verfahren zum Herstellen eines heißen Flüssigfaserpräparats zur enteralen Verabreichung, das eine Beimischung umfasst:
a. eine Portion der rekonstituierbaren Zusammensetzung, umfassend
(i) zwischen 0,1 Gramm und 75 Gramm Pektin;
(ii) unabbaubares Oligosaccharid mit einem Polymerisierungsgrad über 2 und unter 60 Monoseeinheiten; und
(iii) ein Efferveszenzsystem;
und ferner ein Kalziumsalz umfassend, und
b. eine Flüssigkeit mit einer Temperatur, die 35°C übersteigt.

2. Verfahren gemäß Anspruch 1, wobei das unabbaubare Oligosaccharid ausgewählt ist aus der Gruppe bestehend aus Fruktanen, Fruktooligosacchariden, unabbaubaren Dextrinen, Galactooligosacchariden (einschließend Transgalactooligosacchariden), Xylooligosacchariden, Sojabohnenoligosacchariden, Arabinooligosacchariden, Glukooligosacchariden, Mannooligosacchariden, Fucooligosacchariden und Mischungen davon.

3. Verfahren gemäß eines beliebigen der vorhergehenden Ansprüche, wobei die Flüssigkeit ein Volumen zwischen 50 und 1000 ml hat.

4. Verfahren gemäß eines beliebigen der vorhergehenden Ansprüche, wobei die Faser niedrig methoxiliertes Pektin ist.

5. Verfahren gemäß eines beliebigen der vorhergehenden Ansprüche, wobei das Efferveszenzsystem wenigstens eine Base enthält, die Kohlendioxid freisetzt ausgewählt aus einer Gruppe, bestehend aus Natriumkarbonat, Kaliumkarbonat, Natriumbikarbonat, Kaliumbikarbonat, Kalziumbikarbonat, Ammoniumbikarbonat und Natriumglyzinkarbonat.

6. Verfahren gemäß Anspruch 5, des Weiteren einen sauren Bestandteil umfassend, ausgewählt aus der Gruppe bestehend aus Zitronen-, Wein-, Fett-, Fumar-, Apfel-, Milch-, Essig-, Malein- und Benzoesäuren, und Phosphaten.

7. Verfahren gemäß eines beliebigen der vorhergehenden Ansprüche, wobei die rekonstituierbare Zusammensetzung ferner NaCl und/oder Glutamat umfasst.

8. Verfahren gemäß eines beliebigen der vorhergehenden Ansprüche, wobei das daraus resultierende Flüssigfaserprodukt einen pH über 4 hat.

9. Verfahren gemäß eines beliebigen der vorhergehenden Ansprüche, wobei die rekonstituierbare Zusammensetzung ein Kalziumsalz mit einer Löslichkeit unter 0,15 g pro 100 ml Wasser bei 20°C und pH 7 beinhaltet, das mehr als 0,05 Gramm gelöstes Kalzium pro 100 ml Wasser bei einem pH unter 4 und einer Temperatur von 37°C bereitstellt, und das resultierende Flüssigprodukt, (i) eine Viskosität unter 100 mPas hat, und (ii) eine Viskosität von über 250 mPas aufweist, wenn es auf pH 3 angesäuert wird.

10. Heißes Flüssigfaserprodukt mit einer Viskosität unter 100 mPas, einem pH-Wert, der 4 übersteigt und einer Temperatur von wenigstens 37°C, hergestellt mit dem Verfahren gemäß Anspruch 9.

11. Heißes Flüssigfaserprodukt mit einer Viskosität unter 100 mPas, einer Temperatur von mindestens 35°C und einer Viskosität von mindestens 250 mPas, wenn es auf pH 3 angesäuert wird, wobei die Zusammensetzung umfasst (i) ein Kalziumsalz mit einer Löslichkeit unter 0,15 g pro 100 ml Wasser bei 20°C und pH 7, was mehr als 0,05 Gramm gelöstes Kalzium pro 100 ml Wasser bei einem pH unter 4 und einer Temperatur von 37°C bereitstellt, (ii) je Portion zwischen 0,1 Gramm und 75 Gramm Pektin, (iii) unabbaubares Oligosaccharid mit einem Polymerisationsgrad über 2 und unter 60 Monoseeinheiten.

12. Verwendung einer Faser in der Herstellung einer Zusammensetzung zur Verwendung in einem Verfahren für die Behandlung und/oder der Prävention eines ernährungsabhängigen Zustands in einem monogastrischen Säugetier, wobei das Verfahren die enterale Verabreichung an das Säugetier der Zusammensetzung gemäß Anspruch 10 oder 11 einschließt.

13. Verwendung gemäß Anspruch 12, wobei der ernährungsabhängige Zustand Übergewicht ist.

14. Verpackte, rekonstituierbare Zusammensetzung, welche Anweisungen enthält, eine oder mehrere Portionen der rekonstituierbaren Zusammensetzung mit einer Flüssigkeit zu mischen, die eine Temperatur von über 35°C hat, wobei die Zusammensetzung pro Portion umfasst:
(i) zwischen 0,1 Gramm und 75 Gramm Pektin;
(ii) unabbaubares Oligosaccharid mit einem Polymerisierungsgrad über 2 und unter 60 Monoseeinheiten; und
(iii) ein Everveszenzsystem;
(iv) und ein Kalziumsalz.

15. Verpackte Zusammensetzung gemäß Anspruch 14, wobei die Zusammensetzung pro Portion umfasst:
(i) zwischen 0,5 und 15 g niedrig methoxyliertes Pektin und/oder Alginat;
(ii) zwischen 0,01 und 25 Gramm einer Base, die Kohlendioxid freisetzt, vorzugsweise ausgewählt aus einer Gruppe beinhaltend Natriumcarbonat, Kaliumkarbonat, Kalziumkarbonat, Natriumbikarbonat, Kaliumbikarbonat, Kalziumbikarbonat, Ammoniumbikarbonat und Natriumglyzinkarbonat;
(iii) zwischen 0,025 und 5 Gramm eines sauren Bestandteils, vorzugsweise ausgewählt aus der Gruppe einschließlich Zitronen-, Wein-, Fett-, Fumar-, Apfelsäuren, Milch-, Essig-, Malein- und Benzoesäuren; und Phosphaten und
(iv) zwischen 0,05 und 5 Gramm Kalzium.

## Revendications

1. Procédé de préparation d'un produit fibreux liquide chaud pour l'administration entérale, comprenant le mélange:
a. une portion d'une composition apte à être reconstituée comprenant
(i) entre 0,1 gramme et 75 grammes de pectine;
(ii) un oligosaccharide nondigestible avec un degré de polymérisation dépassant 2 et en dessous de 60 unités de monose; et
(iii)un système effervescent;
et comprenant en outre un sel de calcium, et
b. un liquide d'une température qui dépasse 35°C.

2. Procédé selon la revendication 1, dans lequel l'oligosaccharide nondigestible est sélectionné dans le groupe consistant en fructanes, fructooligosaccharides, galactooligosaccharides de dextrines nondigestibles (incluant des transgalactooligosaccharides), xylooligosaccharides, oligosaccharides de soja, arabinooligosaccharides, glucooligosaccharides, mannooligosaccharides, fucooligosaccharides et leurs mélanges.

3. Procédé selon l'une quelconque des revendications précédentes, dans lequel le liquide a un volume compris entre 50 et 1000 ml.

4. Procédé selon l'une quelconque des revendications précédentes, dans lequel la fibre est de la pectine faiblement méthoxylée.

5. Procédé selon l'une quelconque des revendications précédentes, dans lequel le système effervescent contient au moins une base qui libère ledit oxyde de carbone sélectionné dans le groupe consistant en sodium carbonate, potassium carbonate, sodium bicarbonate, potassium bicarbonate, calcium bicarbonates, ammonium bicarbonate et sodium glycine carbonate.

6. Procédé selon la revendication 5, comprenant en outre un ingrédient acidique sélectionné dans le groupe consistant en acides citrique, tartarique, adipique, fumarique, malique, lactique, acétique, maléique et benzoïque, et des phosphates.

7. Procédé selon l'une quelconque des revendications précédentes, dans lequel la composition apte à être reconstituée comprend en outre du NaCl et/ou du glutamate.

8. Procédé selon l'une quelconque des revendications précédentes, dans lequel le produit fibreux liquide obtenu a un pH au-dessus de 4.

9. Procédé selon l'une quelconque des revendications précédentes, dans lequel la composition apte à être reconstituée contient un sel de calcium avec une solubilité inférieure à 0,15 g par 100 ml d'eau à 20°C et un pH 7 qui fournit plus que 0,05 gramme de calcium dissous pour 100 ml d'eau à un pH inférieur à 4 et une température de 37°C, et le produit liquide obtenu (i) a une viscosité inférieure à 100 mPas, et (ii) a une viscosité au-dessus de 250 mPas lorsqu'il est acidifié à un pH 3.

10. Produit fibreux liquide chaud avec une viscosité inférieure à 100 mPas, un pH qui dépasse 4 et une température d'au moins 35°C, préparé selon le procédé en accord avec la revendication 9.

11. Produit fibreux liquide chaud d'une viscosité inférieure à 100 mPas, d'une température d'au moins 35°C et d'une viscosité d'au moins 250 mPas lorsqu'il est acidifié au pH 3, ladite composition comprenant (i) un sel de calcium avec une solubilité inférieure à 0,15 g pour 100 ml d'eau à 20°C et un pH 7 qui fournit plus que 0,05 gramme de calcium dissous pour 100 ml d'eau à un pH inférieur à 4 et une température de 37°C, (ii) par portion entre 0,1 gramme et 75 grammes de pectine, (iii) l'oligosaccharide nondigestible avec un degré de polymérisation supérieur à 2 et inférieur à 60 unités de monose.

12. Utilisation de fibres dans la fabrication d'une composition pour utilisation dans une méthode de traitement et/ou de prévention d'un état réagissant à un régime dans un mammifère monogastrique, ladite méthode comprenant l'administration entérale au mammifère de la composition de la revendication 10 ou 11.

13. Utilisation selon la revendication 12, dans laquelle l'état réagissant au régime est le surpoids.

14. Composition emballée apte à être reconstituée qui comporte des instructions pour mélanger une ou plusieurs portions de la composition apte à être reconstituée avec un liquide d'une température supérieure à 35°C, ladite composition comprenant par portion:
(i) entre 0,1 gramme et 75 grammes de pectine;
(ii) un oligosaccharide nondigestible avec un degré de polymérisation dépassant 2 et en dessous de 60 unités de monose; et
(iii)un système effervescent
(iv) et un sel de calcium.

15. Composition emballée selon la revendication 14, dans laquelle la composition comprend par portion:
(i) entre 0,5 et 15 g de pectine faiblement méthoxylée et/ou d'alginate;
(ii) entre 0,01 et 25 grammes de base qui libère ledit oxyde de carbone, sélectionnée de préférence dans le groupe consistant en sodium carbonate, potassium carbonate, calcium carbonate, sodium bicarbonate, potassium bicarbonate, calcium bicarbonates, ammonium bicarbonate et sodium glycine carbonate;
(iii)entre 0,025 et 5 grammes d'ingrédient acidique, de préférence sélectionné dans le groupe consistant en acides citrique, tartarique, adipique, fumarique, malique, acides lactique, acétique, maléique et benzoïque; et des phosphates; et
(iv) entre 0,05 et 5 grammes de calcium.
